# FASCICULE DE BREVET EUROPEEN

(11) **EP 1 038 860 B1**
(45) Date de publication et mention de la délivrance du brevet: **13.11.2002**
(21) Numéro de dépôt: 00400691.2
(22) Date de dépôt: 13.03.2000
(51) Int. Cl.: C07C 51/38, C07C 59/105

(54) **Procédé de fabrication d'un dérivé acide d'ose par décarboxylation au peroxyde d'hydrogène**
Verfahren zur Herstellung von einem Säurederivat eines Zuckers durch Decarboxylierung mit Wasserstoffperoxid
Process for the production of an acid derivative of an ose by decarboxylation with hydrogen peroxyde

(30) Priorité: 15.03.1999 FR 9903158
(43) Date de publication de la demande: 27.09.2000
(73) Titulaire: Roquette Frères, 62136 Lestrem (FR)
(72) Inventeur: Tamion, Rodolphe, 62157 Allouagne (FR)
(74) Mandataire: Boulinguiez, Didier

(56) Documents cités:
- FR-A- 2 771 413
- ISBELL H. S. ET AL.: "Oxidation of sodium salts of alduronic and glyculosonic acids by sodium peroxide" CARBOHYDRATE RESEARCH, vol. 36, 1974, pages 283-291, XP000856561
- LIANG, YUN TEH S. ET AL: "Synthesis of D-erythroascorbic acid" J. CARBOHYDR. CHEM. (1990), 9(1), 75-84, XP000863157
- HUMPHLETT, WILBERT J.: "Synthesis of some esters and lactones of aldonic acids" CARBOHYDR. RES. (1967), 4(2), 157-64, XP000863144

## Description

La présente invention a pour objet un procédé de fabrication d'un dérivé acide d'ose.

Plus précisément, elle a pour objet un procédé de fabrication d'un dérivé acide d'ose comportant sur la chaîne hydrocarbonée n atomes de carbone, à partir d'un dérivé acide d'ose à n+1 atomes de carbone présentant au moins une fonction α cétone, ou un de ses sels, ce procédé consistant à mettre en contact ledit dérivé acide d'ose à n+1 atomes de carbone avec du peroxyde d'hydrogène dans un milieu réactionnel sans régulation de pH.

Au sens de l'invention, on convient d'entendre par :
- « dérivé acide d'ose comportant au moins une fonction α cétone », un acide mono- ou dicarboxylique dérivé d'un aldose, comportant une fonction cétone en α de la ou des fonction(s) acide(s),
- « aldose », un ose comportant une fonction aldéhyde, en particulier un pentose ou un hexose comportant une telle fonction, de préférence choisi parmi le xylose, l'arabinose, le ribose, le lyxose, le glucose, le mannose, le galactose, le maltose et le lactose.

Le procédé de la présente invention permet en particulier d'obtenir, avec d'excellents rendement et sélectivité, un acide aldonique à partir d'un acide 2-céto-aldonique de rang supérieur.

Les acides aldoniques obtenus par la mise en oeuvre du procédé conforme à l'invention sont d'un grand intérêt en tant que tels, mais sont surtout des intermédiaires de synthèse importants. En effet, une étape complémentaire d'hydrogénation permet d'obtenir facilement les alditols correspondants, qui sont des polyols pouvant être employés dans de multiples applications et notamment en tant que substituts non cariogènes et hypocaloriques du saccharose.

Ces acides aldoniques constituent également une matière première d'intérêt pour préparer des polymères biocompatibles et/ou biodégradables utilisables dans les domaines alimentaire et médical.

C'est en étudiant la méthode déjà ancienne décrite dans le brevet JP 38.15610 de 1963 et reprise par H.S. ISBELL et al. dans l'article de Carbohydrate research, 36, 1974, pages 283 à 291, qui recommande l'utilisation du peroxyde d'hydrogène sur des sels de sodium des acides 2-céto-aldoniques en milieu réactionnel contrôlé pour obtenir des acides aldoniques de rang inférieur que la société Demanderesse a mis au point ce nouveau procédé de fabrication d'un dérivé acide d'ose.

Cette méthode traditionnelle permet de passer, de façon générale, du sel de sodium d'un acide aldonique à n+1 atomes de carbone comportant une fonction cétone en α, à un dérivé acide d'ose à n atomes de carbone, grâce à l'eau oxygénée dans un milieu réactionnel où le pH est maintenu constant par l'addition de soude ou d'acide chlorhydrique.

La conversion du sel de sodium de l'acide 2-céto-gluconique en sel de sodium de l'acide arabinonique peut ainsi être réalisée selon cette méthode.

Cependant la sélectivité de ladite réaction reste relativement faible, car des quantités importantes des sels de l'acide érythronique et de l'acide formique sont coproduits avec ceux de l'acide arabinonique. De plus, la régulation du pH de la réaction conduit à une consommation importante, voire équimolaire, de soude.

Durant une investigation approfondie de cette réaction, la société Demanderesse a découvert que si la réaction est conduite sans régulation de pH, on peut, de façon inattendue, améliorer de manière remarquable la sélectivité de la réaction.

En effet, aucun des documents de l'art antérieur précédemment cités ne décrit, ni ne suggère l'utilisation du peroxyde d'hydrogène sans régulation de pH pour la décarboxylation oxydative d'un dérivé acide d'ose comportant au moins une fonction cétone en α, ou un de ses sels.

Rien en particulier ne suggère qu'il soit possible d'augmenter considérablement la sélectivité de ladite réaction et donc la pureté de l'acide aldonique produit, ce qui se traduit par la diminution significative de la production des co-produits tels que l'acide formique.

Cette nouvelle réaction possède donc un potentiel important et il n'existe pas, à la connaissance de la société demanderesse, de réaction équivalente.

Ainsi, selon l'invention, le procédé de fabrication d'un dérivé acide d'ose comportant sur la chaîne carbonée n atomes de carbone, est caractérisé par le fait que l'on met en contact un dérivé d'ose à n+1 atomes de carbone comportant au moins une fonction α cétone, et/ou un de ses sels, avec du peroxyde d'hydrogène dans un milieu réactionnel sans régulation de pH.

Le procédé conforme à l'invention met donc en oeuvre un dérivé acide d'ose à n+1 atomes de carbone comportant au moins une fonction α cétone, et/ou un de ses sels.

Dans la présente invention, comme indiqué précédemment, on entend par dérivé acide d'ose à n+1 atomes de carbone comportant au moins une fonction α cétone, un acide mono- ou dicarboxylique dérivé d'un aldose présentant au moins une fonction cétone en position α. Cette définition englobe en particulier :
- les acides α-céto-aldoniques, qui sont des acides monocarboxyliques dérivés des aldoses par remplacement du groupement aldéhyde par un groupement carboxy, et le remplacement de la fonction hydroxyle secondaire en position α par une fonction cétone, tels que les acides α-céto-gluconique, α-céto-glucoheptonique, α-cétomannonique, α-céto-idonique, α-céto-gulonique, α-céto-galactonique, α-céto-lyxonique, α-céto-xylonique, α-céto-arabinonique, α-céto-ribonique, α-céto-maltobionique et α-céto-lactobionique, de série D ou L, et en particulier l'acide 2-céto-D-gluconique, l'acide 2,5-dicéto-D-gluconique et l'acide 2-céto-L-gulonique
- les acides α-céto-aldariques, qui sont des acides dicarboxyliques dérivés des aldoses par remplacement des deux groupements terminaux (CHO et CH₂OH) par des groupements carboxy et le remplacement d'au moins une fonction hydroxyle secondaire en position α par une fonction cétone, tels que les acides α-céto-glucarique, α-céto-galactarique, α-céto-arabinarique et α-céto-xylarique.

Dans la présente invention, on entend en particulier par « sel d'un dérivé acide d'ose », un produit choisi dans le groupe comprenant les sels alcalins et alcalino-terreux de tout dérivé acide d'ose tel que défini ci-avant.

Ainsi, par exemple, les sels de calcium et de sodium de ces dérivés acides d'oses conviennent parfaitement. Il peut s'agir notamment des sels de calcium ou de sodium de l'acide 2-céto-gluconique.

Les dérivés acide d'ose à n+1 atomes de carbone comportant au moins une fonction α cétone sont obtenus de manière connue par oxydation des oses ou des dérivés acide d'ose correspondants. Cette étape d'oxydation peut être conduite soit par voie chimique, soit par voie microbiologique.

Dans le cadre de la présente invention, il n'y a aucune contrainte particulière en terme de matière sèche du milieu réactionnel. En pratique, le procédé selon l'invention est caractérisé par le fait que le dérivé acide d'ose à n+1 atomes de carbone et présentant au moins une fonction cétone en α, ou un de ses sels, est apporté en quantité telle que sa concentration dans le milieu réactionnel est comprise entre 1 et 90% en poids, de préférence entre 10 et 50% en poids.

Seules les contraintes de matières sèches minimales sont imposées pour des raisons évidentes d'économie d'évaporation d'eau et de réduction de la taille des réacteurs.

Dans le cas particulier du sel de calcium du dérivé acide d'ose, il est recommandé d'éviter les matières sèches trop élevées. En effet, il est connu que la faible solubilité dudit sel de calcium entraîne une augmentation de la viscosité du milieu réactionnel.

Au dérivé d'acide d'ose à n+1 atomes de carbone comportant au moins une fonction cétone en α, ou un de ses sels, on ajoute lentement, sous agitation, le peroxyde d'hydrogène, de préférence sous forme d'eau oxygénée d'une richesse de 20 à 70%, de préférence de l'ordre de 35%, comme il sera exemplifié ci-après.

Dans un mode préférentiel conforme à l'invention, on ajoute l'eau oxygénée en quantité au plus équimolaire par rapport au dérivé acide d'ose à n+1 atomes de carbone présentant au moins une fonction cétone en α.

En effet, la société Demanderesse a non seulement montré que la réaction de décarboxylation conforme à l'invention ne nécessite pas, contrairement à ce qui est recommandé pour la mise en oeuvre de la méthode de l'art antérieur, d'utiliser un excès d'eau oxygénée, mais également que ledit excès d'eau oxygénée est responsable de la formation parasite d'acide formique et des acides aldoniques de rang inférieur.

Comme il a été mentionné plus haut, une régulation de pH n'est pas non plus réalisée, et la société demanderesse a remarqué à ce sujet, de manière surprenante et inattendue, que la sélectivité de la réaction est nettement améliorée.

De ce fait, et c'est ce qui constitue un avantage important du procédé conforme à l'invention, la réaction ne consomme ni ne génère de sels.

Dans ces conditions, il est à noter qu'aucune production de chlorures ou de carbonates de sodium ou de calcium ne sera déplorée lors de la réaction.

Le procédé conforme à l'invention est donc mis en oeuvre sans régulation de pH, quelle que soit la forme du dérivé acide d'ose introduit dans le milieu réactionnel.

Le procédé de l'invention est également mis en oeuvre à une température comprise entre 0 et 100°C, de préférence comprise entre 20 et 90°C.

Dans le cas particulier où l'on choisit de décarboxyler la forme acide du dérivé acide d'ose à n+1 atomes de carbone présentant au moins une fonction α cétone, il est recommandé de chauffer le milieu réactionnel, car la réaction est plus lente.

Cependant, on préfère avantageusement mettre en oeuvre la décarboxylation des sels des dérivés acides d'ose. En effet, cette réaction étant exothermique, il est avantageusement recommandé d'utiliser cette énergie dégagée pour assurer le chauffage du milieu réactionnel.

Ce résultat constitue donc un autre avantage du procédé conforme à l'invention, car aucune dépense énergétique extérieure au système n'est requise, ce qui représente alors un gain économique appréciable du point de vue industriel.

La régulation de la température n'est donc pas un facteur limitant. On peut ainsi procéder au démarrage de la réaction à température ambiante, et lorsque, par addition d'eau oxygénée, la température du milieu réactionnel atteint environ 40°C, la maintenir à cette valeur par refroidissement, par tout moyen connu en soi.

Cependant, il a été observé que cette régulation de température ne présente pas de nécessité absolue, car ni le rendement ni la sélectivité ne sont significativement affectés par l'élévation thermique incontrôlée du milieu réactionnel.

Il est toutefois généralement admis que des températures supérieures à 90°C, outre qu'elles nécessitent l'emploi de réacteurs résistant à la pression, peuvent conduire à une dégradation des produits de la réaction.

L'isolement des produits de la réaction s'effectue par simple filtration, dans le cas où l'on choisit le sel de calcium du dérivé acide d'ose car ce dernier présente l'avantage de précipiter naturellement dans le milieu réactionnel, ou par traitement au noir afin de décolorer et éliminer l'eau oxygénée résiduelle éventuelle, dans le cas où l'on choisit le sel de sodium du dérivé acide d'ose.

On réalise enfin avantageusement la concentration du milieu réactionnel par tout moyen connu en soi, afin de favoriser la cristallisation spontanée du produit de la réaction.

L'invention sera mieux comprise au moyen des exemples qui suivent et qui ont pour seul but de mieux illustrer l'invention sans vouloir la réduire aux modes de réalisation expressément décrits et au dérivé acide d'ose mis en oeuvre.

### EXEMPLE 1

1500 g de 2-céto-gluconate de sodium (6,94 moles) sont dissous dans 31 d'eau. L'eau oxygénée à 35% w/w (674g, 6,94 moles) est additionnée pendant 2 heures et sous agitation.

La température de la réaction augmente progressivement de 1°C/min et lorsqu'elle atteint 40°C, on refroidit le milieu réactionnel afin de le maintenir à cette valeur.

La réaction est poursuivie pendant 3 heures supplémentaires, ce qui correspond au temps nécessaire pour que la concentration en peroxyde soit inférieure à 25 ppm.

On observe un dégagement gazeux de dioxyde de carbone pendant les premières heures de la réaction, et un contrôle du bon déroulement de la réaction est effectuée par la mesure du taux de sucres réducteurs par la méthode de Bertrand, à une valeur finale inférieure à 1% sec/sec.

Après la réaction, le milieu réactionnel est traité au noir pendant 1 heure à 20°C, ce qui permet de décolorer le milieu réactionnel et de détruire les 10 à 25 ppm de peroxydes résiduels.

Après filtration, la solution est concentrée jusqu'à une concentration de 600g/l, valeur à laquelle l'arabinonate cristallise spontanément.

Le taux de conversion est de 100%, et le rendement final en arabinonate de sodium est de 98%. On ne déplore aucune production significative de sels de sodium de l'acide formique et de l'acide érythronique.

### EXEMPLE 2

1670g de 2-céto-gluconate de calcium (3,47 moles, contenant 11,6% d'eau) sont mis en solution dans 31 d'eau. L'eau oxygénée à 35% w/w (674,5 g, 6,94 moles) est additionnée en 2 heures sous agitation. La température de la réaction augmente progressivement de 1°C/min et lorsqu'elle atteint 40°C, on refroidit le milieu réactionnel afin de le maintenir à cette valeur.

La réaction est poursuivie pendant 3 heures supplémentaires, ce qui correspond au temps nécessaire pour que la concentration en peroxydes soit inférieure à 25 ppm.

On observe un dégagement gazeux de dioxyde de carbone pendant les premières heures de la réaction.

Le 2-céto-gluconate de calcium se dissolvant progressivement au cours de l'addition d'eau oxygénée, le milieu devient alors limpide.

L'arabinonate de calcium précipite lorsque 80% de l'eau oxygénée ont été introduits dans le milieu réactionnel. A ce stade, on contrôle le bon déroulement par la mesure du taux de sucres réducteurs par la méthode de Bertrand à une valeur finale inférieure à 1 % sur sec.

Le milieu réactionnel est alors refroidi à 20°C.

Après filtration, le solide est séché. Le taux de conversion est de 100%. Le rendement en arabinonate de calcium après cristallisation est de 93%. Le produit cristallise avec 5 molécules d'eau. On ne déplore également aucune production significative de sels de sodium de l'acide formique et de l'acide érythronique.

### EXEMPLE 3

Les essais 2 à 6 suivants consistent à préparer des dérivés acide d'ose selon le procédé conforme à l'invention à partir des sels de l'acide 2-céto-gluconique, mais en modifiant les conditions opératoires décrites dans les exemples 1 et 2 précédents.

Les produits obtenus en fin de réaction présentent les caractéristiques énoncées dans le tableau ci-dessous et sont comparés à ceux préparés par la méthode de l'art antérieur (Essai n°1).

| **ESSAIS** | **1** | **2** | **3** | **4** | **5** | **6** |
|---|---|---|---|---|---|---|
| Nature du sel de l'acide 2 céto gluconique | Sodium | Sodium | Calcium | Calcium | Calcium | Calcium |
| Quantités H₂O₂ (Equivalents) | 1,1 | 1 | 1,1 | 1 | 1,1 | 1 |
| Température (°C) | 65-70 | 65-70 | 65-70 | 40 | 40 | 40 |
| pH régulé à 7 | Oui | Non | Non | Non | Non | Non |
| Matière sèche (%) | 10 | 10 | 10 | 10 | 20 | 30 |
| Soude consomée (Equivalents) | 0,94 | 0 | 0 | 0 | 0 | 0 |
| Taux de conversion (%) | 100 | 100 | 100 | 100 | 100 | 100 |
| Sels d'arabinonate (%*) | 91,4 | 98,5 | 97,5 | 99,3 | 98,3 | 99,3 |
| Sels d'érythronate (%*) | 2,5 | 0 | 0 | 0 | 0 | 0 |
| Sels de formiate (%*) | 6,1 | 1,5 | 2,5 | 0,7 | 1,7 | 0,7 |

| | | | | | | |
|---|---|---|---|---|---|---|
| * : rendement molaire | | | | | | |

Les résultats montrent clairement que l'absence de régulation de pH dans le procédé conforme à l'invention permet d'atteindre une meilleure sélectivité de la réaction que celle obtenue avec la méthode conduite en conditions contrôlées.

Les rendements de production d'arabinonate à partir de 2-céto-gluconate sont en outre supérieurs à 98,5% et ce, quelle que soit la température mise en oeuvre, dès que l'on conduit la réaction sans régulation de pH et en introduisant également une quantité équimolaire d'eau oxygénée. Ainsi, aucune production significative de coproduits de la réaction n'est déplorée, et l'on peut noter l'absence de formation d'acide érythronique.

Il apparaît clairement que le procédé conforme à l'invention permet d'obtenir l'arabinonate à partir d'acide 2-céto-gluconate avec un rendement et une sélectivité encore jamais atteints.

### EXEMPLE 4

Dans cet exemple, on met en oeuvre un mode opératoire identique à celui décrit dans l'exemple 1 si ce n'est que le 2-céto-gluconate de sodium est remplacé, poids pour poids, par du 2-céto-gulonate de sodium.

Dans ces conditions, on obtient également un taux de conversion de 100%. Le rendement en xylonate de sodium est de 98,5%.

## Revendications

1. Procédé de fabrication d'un dérivé acide d'ose comportant sur la chaîne carbonée n atomes de carbone, **caractérisé par le fait que** l'on met en contact un dérivé acide d'ose à n+1 atomes de carbone comportant au moins une fonction α cétone, et/ou un de ses sels, avec du peroxyde d'hydrogène dans un milieu réactionnel sans régulation de pH.

2. Procédé de fabrication selon la revendication 1, **caractérisé par le fait que** le dérivé acide d'ose comportant au moins une fonction α cétone ou un de ses sels est apporté en quantité telle que sa concentration dans le milieu réactionnel soit comprise entre 1 1 et 90% en poids, de préférence entre 10 et 50% en poids.

3. Procédé de fabrication selon l'une ou l'autre des revendications 1 et 2 , **caractérisé par le fait que** l'on utilise du peroxyde d'hydrogène, de préférence sous forme d'eau oxygénée, d'une richesse de 20 à 70%, de préférence de l'ordre de 35%.

4. Procédé de fabrication selon l'une ou l'autre des revendications 1 à 3, **caractérisé par le fait que** l'on introduit l'eau oxygénée dans le milieu réactionnel en quantité au plus équimolaire par rapport audit dérivé acide d'ose à n+1 atomes de carbone comportant au moins une fonction α cétone.

5. Procédé de fabrication selon l'une quelconque des revendications 1 à 4, **caractérisé par le fait que** l'on travaille à une température comprise entre 0 et 100°C, de préférence comprise entre 20 et 90°C.

6. Procédé de fabrication selon l'une quelconque des revendications 1 à 5, **caractérisé par le fait que** le dérivé acide d'ose à n+1 atomes de carbone comportant au moins une fonction α cétone est choisi dans le groupe constitué des acides 2-céto-D-gluconique, 2,5-dicéto-D-gluconique et 2-céto-L-gulonique et/ou l'un de ses sels de calcium ou de sodium.

## Patentansprüche

1. Verfahren zur Herstellung eines Osesäurederivats, das in der kohlenstoffhaltigen Kette n Kohlenstoffatome aufweist, **dadurch gekennzeichnet, dass** man ein Osesäurederivat mit n+1 Kohlenstoffatomen, das mindestens eine α-Cetonfunktion aufweist, und/oder eines seiner Salze mit Wasserstoffperoxid in einem Reaktionsmedium ohne pH-Regulierung in Kontakt bringt.

2. Verfahren nach Anspruch 1, **dadurch gekennzeichnet, dass** das Osesäurederivat mit mindestens einer α-Cetonfunktion oder eines seiner Salze in einer solchen Menge zugeführt wird, dass seine Konzentration im Reaktionsmedium zwischen 1 und 90 Gew.-%, vorzugsweise zwischen 10 und 50 Gew.-%, beträgt.

3. Verfahren nach einem der Ansprüche 1 und 2, **dadurch gekennzeichnet, dass** man Wasserstoffperoxid vorzugsweise in Form von Wasserstoffperoxid mit einem Gehalt von 20 bis 70%, vorzugsweise etwa 35%, verwendet.

4. Verfahren nach einem der Ansprüche 1 bis 3, **dadurch gekennzeichnet, dass** man Wasserstoffperoxid in einer höchstens äquimolaren Menge bezüglich des Osesäurederivats mit n+1 Kohlenstoffatomen, das mindestens eine α-Cetonfunktion aufweist, in das Reaktionsmedium einführt.

5. Verfahren nach einem der Ansprüche 1 bis 4, **dadurch gekennzeichnet, dass** man bei einer Temperatur zwischen 0 und 100°C, vorzugsweise zwischen 20 und 90°C, arbeitet.

6. Verfahren nach einem der Ansprüche 1 bis 5, **dadurch gekennzeichnet, dass** das Osesäurederivat mit n+1 Kohlenstoffatomen, das mindestens eine α-Cetonfunktion aufweist, aus der Gruppe ausgewählt ist, die aus 2-Ceto-D-gluconsäure, 2,5-Diceto-Dgluconsäure und 2-Ceto-L-gulonsäure und/oder einem ihrer Calcium- oder Natriumsalze besteht.

## Claims

1. A process for manufacturing an acid derivative of ose containing n carbon atoms on the carbonic chain, **characterized by** the fact that an acid derivative of an ose with n+1 carbon atoms containing at least one α ketone function, and/or one of its salts, is brought into contact with hydrogen peroxide in a reaction medium without pH regulation.

2. Process of manufacture according to claim 1, **characterized by** the fact that the acid derivative of ose containing at least one α ketone function or one of its salts is provided in a quantity such that its concentration in the reaction medium is between 1% and 90% by weight, preferably between 10 and 50% by weight.

3. Process of manufacture according to either one of claims 1 and 2, **characterized by** the fact that hydrogen peroxide is used, preferably in the form of oxygenated water, of a strength of 20 to 70%, preferably of the order of 35%.

4. Process of manufacture according to any one of claims 1 to 3, **characterized by** the fact that oxygenated water is introduced into the reaction medium in a quantity at most equimolar with respect to said acid derivative of ose with n+1 carbon atoms containing at least one α ketone function.

5. Process of manufacture according to any one of claims 1 to 4, **characterised by** the fact that it is performed at a temperature of between 0 and 100°C, preferably between 20 and 90°C.

6. Process of manufacture according to any one of claims 1 to 5, **characterised by** the fact that the acid derivative of ose with n+1 carbon atoms containing at least one α ketone function is chosen from the group constituted by 2-keto-D-gluconic, 2,5-diketo-D-gluconic and 2-keto-L-gluconic acids and/or one of its calcium or sodium salts.
